# EUROPÄISCHE PATENTSCHRIFT

(11) **EP 2 218 412 B1**
(45) Veröffentlichungstag und Bekanntmachung des Hinweises auf die Patenterteilung: **03.07.2013**
(21) Anmeldenummer: 10153492.3
(22) Anmeldetag: 12.02.2010
(51) Int. Cl.: A61B 17/32, A61M 1/00, A61B 17/16

(54) **Medizinisches Instrument zum Schneiden von Gewebe**
Medical instrument for cutting tissue
Instrument médical destiné à couper des tissus

(30) Priorität: 16.02.2009 DE 102009010561
(43) Veröffentlichungstag der Anmeldung: 18.08.2010
(73) Patentinhaber: Karl Storz GmbH & Co. KG, 78532 Tuttlingen (DE)
(72) Erfinder: Berberich, Sascha, 78532, Tuttlingen (DE)
(74) Vertreter: Witte, Weller & Partner

(56) Entgegenhaltungen:
- EP-A1- 1 882 455
- WO-A1-92/15255
- US-A- 4 775 365
- US-A- 6 053 928
- US-A- 6 156 049
- US-A1- 2003 125 639

## Beschreibung

Die Erfindung betrifft ein medizinisches Instrument zum Schneiden von Gewebe, mit einem rohrförmigen Außenschaft, der im Bereich seines distalen Endes zumindest eine Außenschaftöffnung mit zumindest einer Außenschaftschneide aufweist, und mit einem rohrförmigen, um eine Rotationsachse rotierbaren, in dem Außenschaft aufgenommenen Innenschaft, der im Bereich seines distalen Endes zumindest eine Innenschaftöffnung mit zumindest einer Innenschaftschneide aufweist, die bei in Rotation versetztem Innenschaft mit der zumindest einen Außenschaftschneide des Außenschaftes schneidend zusammenwirkt, wobei die zumindest eine Innenschaftöffnung in einer parallel zur Rotationsachse verlaufenden Projektionsebene eine gekrümmte Längsachse aufweist.

Ein derartiges Instrument ist aus der US 2003/0125639 A1 bekannt.

Weitere derartige medizinische Instrumente sind aus der US 6 156 049 A und der US 4 775 365 A sowie der WO 92/15255 A1 bekannt.

Aus der US 2003/0125639 A1 ist eine Biopsienadel bekannt, die einen Außenschaft aufweist, der mit einer geschlossenen pyramidenartigen Kappe versehen ist. In dem Außenschaft ist eine sich in axialer Richtung erstreckende Außenschaftöffnung vorhanden. Im Außenschaft ist ein hohlzylindrischer Innenschaft drehbar aufgenommen. In der Wand des Innenschafts ist eine Öffnung vorhanden, die in einer parallel zur Rotationsachse verlaufenden Projektionsebene einen gekrümmten Verlauf aufweist.

Aus der US 6 156 049 A ist eine Vorrichtung zur transurethralen Resektion der Prostata bekannt, bei der der Außenschaft eine oder mehrere Außenschaftöffnungen aufweist. Es sind Innenschäfte dargestellt, die ebenfalls mehrere Innenschaftöffnungen aufweisen, wobei die Geometrie der Innenschaftöffnungen stark variiert und wobei auch Ausführungen angegeben sind, die gegenüber der Mittellängsachse eine geneigte Längsachse aufweisen können.

Aus der US 4 775 365 A ist eine Kanüle zur Fettabsaugung bekannt, bei der im Außenschaft eine geradlinige, längs verlaufende Außenschaftöffnung vorhanden ist. Im rotierenden Innenschaft ist eine einzige, sich schraubenlinienförmig um die Rotationsachse windende Außenschaftöffnung vorhanden.

Aus der WO 92/15255 A1 ist eine Vorrichtung zum Entnehmen von sowohl harten, Materialien, also insbesondere Knochenmaterialien, als auch von weichen Materialien, wie Gewebe aus Lebewesen, bekannt. Der drehbare Innenschaft weist dabei in seinem distalen Endbereich sechs Innenschaftöffnungen auf, deren Mittellängsachse sich längs der Rotationsachse erstreckt. Die Seitenflanken bzw. Innenschaftschneiden sind seitlich bauchig ausgeweitet.

Aus der US 6 053 928 A1 ist ein medizinisches Instrument zum Schneiden von Gewebe bekannt, bei dem die Innenschaftschneide der Innenschaftöffnungen einen keilförmigen Querschnitt mit einer Keilspitze aufweist, welche an einer Außenfläche des Innenschaftes angeordnet ist. Ferner weist die Außenschaftschneide des Außenschafts einen keilförmigen Querschnitt auf, dessen Keilspitze an der Innenfläche des Außenschaftes angeordnet ist.

Aus der EP 1 882 455 A1 ist ein medizinisches Instrument zum Schneiden von Gewebe bekannt, bei dem der Innenschaft drei unterschiedlich ausgeformte, d.h. mit unterschiedlicher Breite versehene, Innenschaftöffnungen aufweist, die mit einer oder mehreren Außenschaftöffnungen des Außenschaftes zusammenwirken können.

Solche Instrumente werden in der minimalinvasiven Chirurgie zum Abtrennen von Gewebe im menschlichen oder tierischen Körper verwendet. Dazu wird das distale Ende des Schaftes zu dem Operationsgebiet geführt, in dem sich das abzutrennende Gewebe befindet. Zum Abtrennen des Gewebes wird das Schneidelement mittels eines externen oder internen Motors in Rotation versetzt. Die an dem Schneidelement ausgebildete Schneide wirkt während des Umlaufens mit einem als Schneide ausgebildeten Rand der Außenschaftöffnung schneidend zusammen, indem die Schneide des Schneidelements an der Schneide der Außenschaftöffnung bei jedem Umlauf vorbeiläuft. Um das abzutrennende Gewebe zwischen die zusammenwirkenden Schneiden zu bringen, ist bei solchen Instrumenten der Schaft mit einer Saugquelle verbunden, deren Saugwirkung durch den Schaft bis zur Außenschaftöffnung reicht, um das abzutrennende Gewebe durch diese Öffnung in den Schaft zu saugen, damit die Schneiden das Gewebe abtrennen können. Durch den Unterdruck wird das abgetrennte Gewebe durch den Schaft abgesaugt.

Das aus der EP 1 882 455 A1 bekannte Instrument weist einen Außenschaft auf, der an seinem distalen Ende eine dreieck- bzw. ovalförmige, mit einer Schneide versehene Öffnung aufweist. In dem Außenschaft ist ein rohrförmiger rotierbarer Innenschaft aufgenommen, an dessen distalem Ende ein Schneidelement ausgebildet ist. Das Schneidelement weist mehrere ovalförmige, ebenfalls mit einer Schneide versehene Öffnungen auf, die, in einer Umfangsrichtung gesehen, unterschiedlich breit sind.

Das abzutrennende Gewebe wird in eine der Öffnungen des in dem Außenschaft rotierenden Schneidelements gesaugt. Das Gewebe wird danach abgetrennt, indem die als Schneide ausgebildete Kante der Öffnung des Schneidelements, in der das abzutrennende Gewebe gesaugt ist, an der in Umlaufrichtung vorderen Kante der Öffnung des Außenschafts vorbeiläuft. Nachdem das Gewebe abgetrennt ist, wird es durch den Innenschaft zum proximalen Ende des Instruments hin abgesaugt.

Dadurch, dass die mit der Schneide des Außenschafts schneidend zusammenwirkende Schneide des Schneidelements einen konvexen, d.h. nach außen gewölbten Verlauf aufweist, stellt man fest, dass sich dichtes, zähes Gewebe mittig zwischen den beiden Schneiden ansammelt und erst am Ende einer Schnittphase tatsächlich abgeschnitten wird, wodurch es oft zu Verstopfungen kommt. Der Grund hierfür ist, dass dieses Gewebe aufgrund des von Beginn an und während eines Großteils der Schnittphase wirkenden ungünstigen Schnittwinkels nicht abgetrennt, sondern mittig zwischen den beiden Schneiden "gedrängt" wird, und erst am Ende der Schnittphase innerhalb kurzer Zeit zerkleinert wird. Die hohe benötigte Schnittkraft während der kurzen Schnittzeit führt zu einer deutlichen Erhöhung der aufzuwendenden Schnittleistung am Ende der Schnittphase, wodurch eine ungleichförmige Belastung des Schneidelements und somit eine gestörte Laufruhe resultiert.

Somit besteht der Nachteil des bekannten Instruments darin, dass das abzutrennende Gewebe nicht sehr effizient, effektiv und einwandfrei abgetrennt werden kann.

Es ist daher Aufgabe der vorliegenden Erfindung ein Instrument der eingangs genannten Art dahingehend weiterzubilden, dass die Schnittleistung bzw. Schnittwirkung verbessert wird.

Erfindungsgemäß wird die Aufgabe dadurch gelöst, dass der Innenschaft drei umfänglich versetzt angeordnete Innenschaftöffnungen aufweist, wobei die Breite der Innenschaftöffnungen, in einer Umfangsrichtung gesehen, geringer ist als der Abstand zwischen den Innenschaftöffnungen, und wobei sich die drei Innenschaftöffnungen bis zu einer distalen Endfläche erstrecken, wobei diese im Bereich der distalen Endfläche neben einem distalen Endpunkt verlaufen.

Das Vorsehen von drei umfänglich versetzt angeordneten Innenschaftöffnungen hat den Vorteil, dass während einer vollen Umdrehung des Innenschaftes drei Schneidevorgänge erfolgen können. Somit wird eine sehr gute Effizienz der Schnittleistung und der Schnittwirkung erzielt. Zudem kann, je nach Anwendungsbereich und Gewebekonsistenz, d.h. je nach benötigter Schnittaggressivität, ein Instrument mit entsprechendem Schneidwinkel eingesetzt werden, um eine maximale Schnitteffizienz zu ermöglichen.

Die Maßnahme, dass die Breite der Innenschaftöffnungen geringer als der Abstand zwischen den Innenschaftöffnungen ist, trägt weiter zur Stabilität und Steifigkeit des Innenschaftes am distalen Ende bei.

Dadurch, dass sich die Innenschaftöffnungen bis zu einer distalen Endfläche erstrecken, wird die Einsatzbreite weiter dahingehend erhöht, dass auch Gewebe, welches sich im Bereich des distalen Endes des Außenschaftes befindet und somit nur schwer erreichbar ist, angesaugt und abgetrennt werden kann.

Bei den nunmehr vorgesehenen drei Innenschaftöffnungen trägt die Maßnahme, dass diese quer zu einem distalen Endpunkt verlaufen, dazu bei, dass sich die Innenschaftöffnungen nicht schneiden und auch nicht auf den gleichen Punkt zulaufen, wodurch die Stabilität und Steifigkeit des Innenschaftes im distalen Endbereich erhöht wird.

In einer weiteren Ausgestaltung der Erfindung weist die Längsachse der zumindest einen Innenschaftöffnung einen kreisbogenförmigen Verlauf auf.

Diese Maßnahme hat den Vorteil, dass ein stetiger, kontinuierlicher Schnittverlauf resultiert, wodurch die Schnittleistung bzw. Schnittwirkung verbessert und die Laufruhe des rotierenden Innenschafts begünstigt wird.

In einer weiteren Ausgestaltung der Erfindung weist die zumindest eine Innenschaftschneide der zumindest einen Innenschaftöffnung einen keilförmigen Querschnitt mit einer Keilspitze auf, welche an einer Außenfläche des Innenschafts angeordnet ist.

Diese Maßnahme hat den Vorteil, dass durch die zugespitzt verlaufende Innenschaftschneide die Schnittcharakteristik und die Schnittwirkung des erfindungsgemäßen Instruments weiter verbessert werden. Zudem kann je nach Anwendungsbereich und Gewebekonsistenz ein Instrument mit entsprechendem Schneidwinkel eingesetzt werden, um eine maximale Schnitteffizienz zu erreichen.

In einer weiteren Ausgestaltung der Erfindung weist die zumindest eine Außenschaftschneide der zumindest einen Außenschaftöffnung einen keilförmigen Querschnitt mit einer Keilspitze auf, welche an einer Innenfläche des Außenschafts angeordnet ist.

Diese Maßnahme hat den Vorteil, dass durch die zugespitzt verlaufende Außenschaftschneide die Schnittcharakteristik und die Schnittwirkung des erfindungsgemäßen Instruments weiter verbessert werden. Zudem kann, je nach Anwendungsbereich und Gewebekonsistenz, ein Instrument mit entsprechendem Schneidwinkel eingesetzt werden, um eine maximale Schnitteffizienz zu erreichen.

In einer weiteren Ausgestaltung der Erfindung weist der Außenschaft drei umfänglich versetzt angeordnete Außenschaftöffnungen auf.

Diese Maßnahme hat den Vorteil, dass die Schnittleistung des erfindungsgemäßen Instruments weiter verbessert werden kann. Ein Außenschaft mit drei Außenschaftöffnungen kann mit einem Innenschaft mit drei Innenschaftöffnungen kombiniert werden, um die Schnittleistung des erfindungsgemäßen Instruments noch weiter zu erhöhen. Dadurch wird erreicht, dass wesentlich mehr Schneiden im Einsatz sind und dadurch mehr Schneidvorgänge realisiert werden, und somit auch mehr Gewebe in kurzer Zeit abgetrennt werden kann.

In einer weiteren Ausgestaltung der Erfindung ist der Innenschaft mittels eines Motors in beide Umlaufrichtungen bewegbar.

Da entsprechend der Umlaufrichtung unterschiedliche Schneidepaare zusammenwirken, ändert sich auch der Schnittverlauf und somit die Schnittaggressivität des Instruments. Diese Maßnahme hat folglich den Vorteil, dass je nach Anwendungsbereich und Gewebekonsistenz, d.h. je nach benötigter Schnittaggressivität, eine entsprechende Umlaufrichtung gewählt werden kann, um eine maximale Schnitteffizienz zu erreichen.

In einer weiteren Ausgestaltung der Erfindung ist der Innenschaft über einen vordefinierten Winkelbereich oszillierend bewegbar.

Diese Maßnahme hat den Vorteil, dass zum einen zwei unterschiedliche Schnittverläufe unterschiedlicher Schnittaggressivität periodisch aufeinanderfolgend durchgeführt werden können, und zum anderen, dass auch die Gefahr eines Verstopfens des Innenschafts weiter reduziert wird, da das Risiko von haftenden Gewebestückchen durch die "schüttelnde" Bewegung minimiert wird. Zudem arbeiten bei einer derartigen Ausgestaltung des erfindungsgemäßen Instruments die Schneiden noch effizienter, wodurch mehr Gewebe schneller abgetrennt werden kann.

In einer weiteren Ausgestaltung der Erfindung wird bei jeder Auslenkung des oszillierenden Innenschafts ein Schnittvorgang durchgeführt.

Diese Maßnahme hat den Vorteil, dass die Auslenkung minimiert und die Anzahl der durchgeführten Schnittvorgänge pro Zeit maximiert wird.

Es versteht sich, dass die vorstehend genannten und die nachstehend noch zu erläuternden Merkmale nicht nur in den angegebenen Kombinationen, sondern auch in anderen Kombinationen einsetzbar sind, ohne den Rahmen der vorliegenden Erfindung zu verlassen.

Die Erfindung wird nachfolgend anhand eines ausgewählten Ausführungsbeispiels in Zusammenhang mit den Zeichnungen näher beschrieben und erläutert. Es zeigen:
- Fig. 1: ein medizinisches Instrument zum Schneiden von Gewebe in teil- weise geschnittener Seitenansicht,
- Fig. 2: eine vergrößerte Darstellung des distalen Endabschnitts des In- struments von Fig. 1,
- Fig. 3: eine perspektivische Darstellung des distalen Endabschnitts des In- nenschafts mit drei Innenschaftöffnungen,
- Fig. 4: einen Schnitt entlang der Schnittlinie A-A in Fig. 2,
- Fig. 5A-C: einen Schnittvorgang mit drei unterschiedlichen Positionen der Innenschaftöffnung gemäß dem Ausführungsbeispiel von Fig. 1 und Fig. 2, und
- Fig. 6A-C: einen Schnittvorgang mit drei unterschiedlichen Positionen der Innenschaftöffnung gemäß einem weiteren Ausführungsbeispiel.

Ein in den Figuren dargestelltes medizinisches Instrument zum Schneiden von Gewebe ist in seiner Gesamtheit mit der Bezugsziffer 10 bezeichnet.

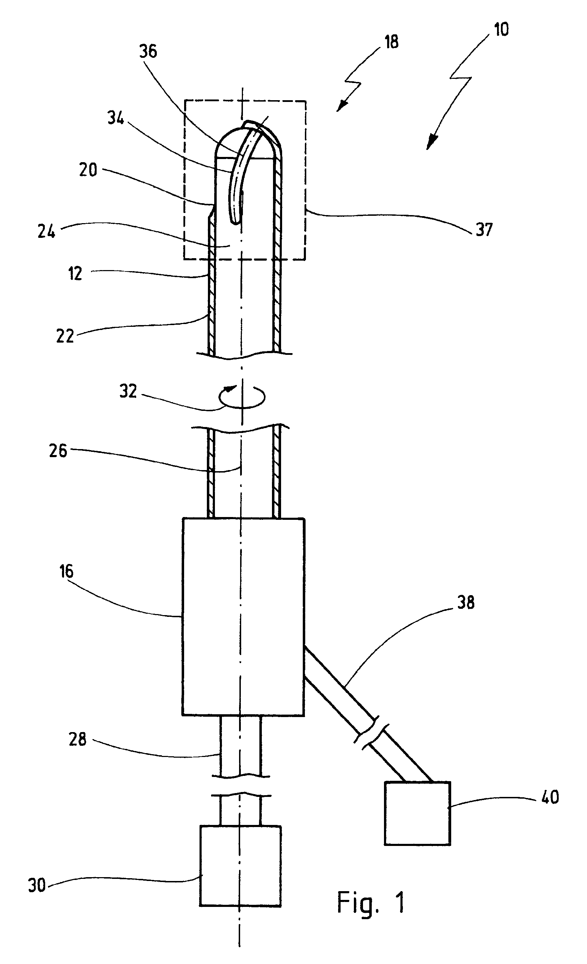

Das in Fig 1,2 und 4 dargestellte Ausführungsbeispiel zeigt nur eine von drei Innenschaftöffnungen, um die prinzipielle Ausgestaltung einer Innenschaftöffnung zu erläutern.

Das medizinische Instrument 10 weist einen rohrförmigen Außenschaft 12 auf, der an seinem proximalen Ende mit einem Gehäuse 16 verbunden ist.

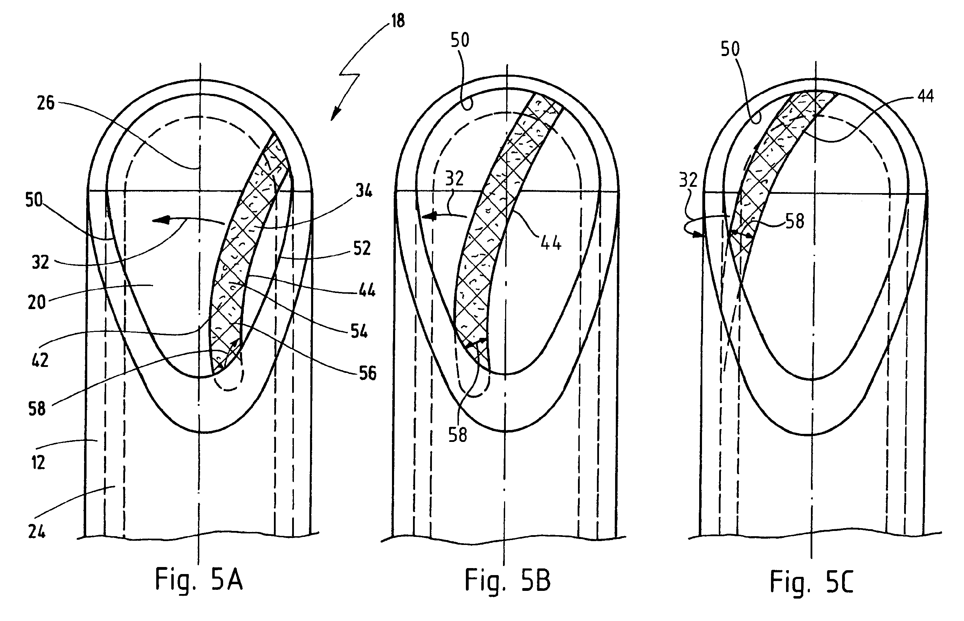

In seinem abgerundeten und geschlossenen distalen Ende 18 weist der Außenschaft 12 eine Außenschaftöffnung 20 auf. Die Außenschaftöffnung 20 ist dadurch gebildet, dass in einer Wand 22 des Außenschaftes 12 eine umfänglich und axial begrenzte, etwa ovalförmige Öffnung ausgebildet ist, wie aus Fig. 1 in Verbindung mit Fig. 5 hervorgeht.

Ein rohrförmiger Innenschaft 24 ist in dem Außenschaft 12 um eine Rotationsachse 26 rotierbar aufgenommen und an dem proximalen Ende über eine Antriebswelle 28 mit einem Motor 30 verbunden. Die Antriebswelle 28 wird über den Motor 30 in Rotation gemäß dem Rotationspfeil 32 versetzt, wobei die Drehbewegung der Antriebswelle 28 auf den Innenschaft 24 übertragen wird, welcher bezüglich des ruhenden Außenschafts 12 in Rotation versetzt wird.

Es sei zudem anzumerken, dass der Motor 30 auch derart ausgebildet sein kann, dass der Innenschaft 24 zusätzlich in die zum Rotationspfeil 32 entgegengesetzte Richtung bzw. oszillierend in beide Richtungen bewegt werden kann.

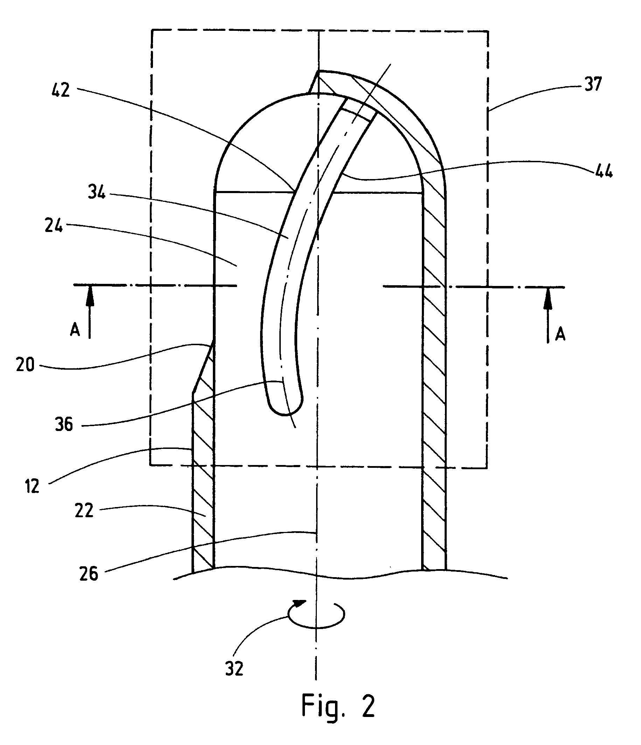

Der Innenschaft 24 weist an dem distalen Ende 18, im Bereich der Außenschaftöffnung 20 des Außenschafts 12, eine Innenschaftöffnung 34 auf. Die Innenschaftöffnung 34 erstreckt sich entlang einer gekrümmten Längsachse 36 und weist eine längliche, säbelförmige Form auf, wie aus Fig. 2 näher ersichtlich ist. Der gekrümmte Verlauf der Längsachse 36 ergibt sich dabei durch Projektion der Längsachse 36 auf eine parallel zur Rotationsachse 26 verlaufenden Projektionsebene 37.

Ferner ist das Instrument 10 über einem Saugstutzen 38 des Gehäuses 16 an eine Saugquelle 40 angeschlossen. Bei eingeschalteter Saugquelle 40 bildet sich ein Saugstrom durch den Innenschaft 24 bis zur Innenschaftöffnung 34, der von der Innenschaftöffnung 34 zum Saugstutzen 38 gerichtet ist.

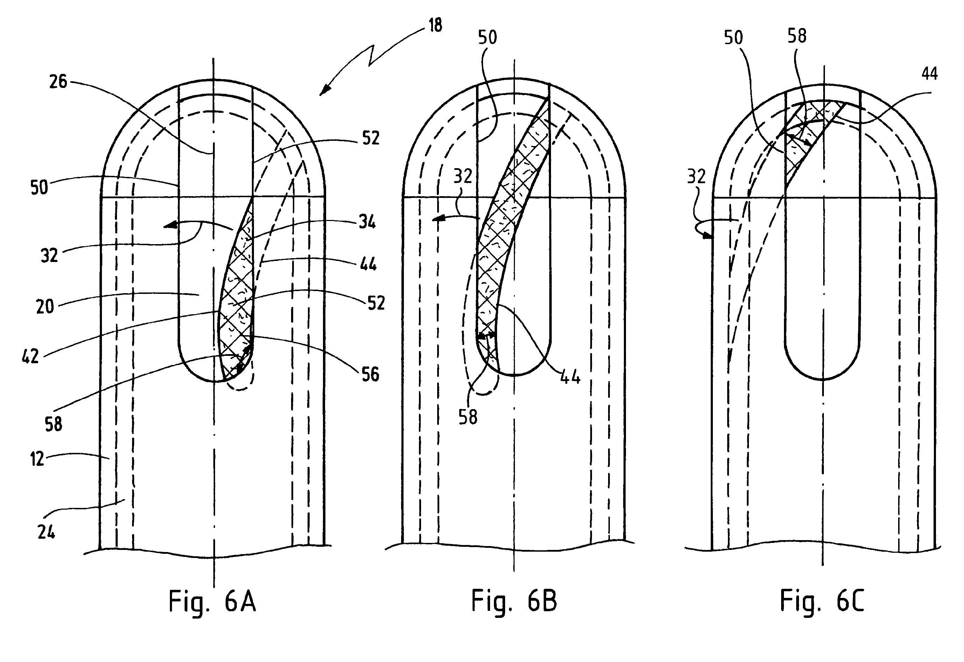

Wie aus der vergrößerten Darstellung von Fig. 2 weiter ersichtlicht ist, weist der Innenschaft 24 eine erste, in Umlaufrichtung vordere Innenschaftschneide 42 und eine zweite, in Umlaufrichtung hintere Innenschaftschneide 44 auf. Im dargestellten Ausführungsbeispiel wirkt die zweite Innenschaftkante 44 schneidend mit dem Außenschaft 12 zusammen, wie nachfolgend bei der Beschreibung der Fig. 5 und 6 detailliert erläutert wird.

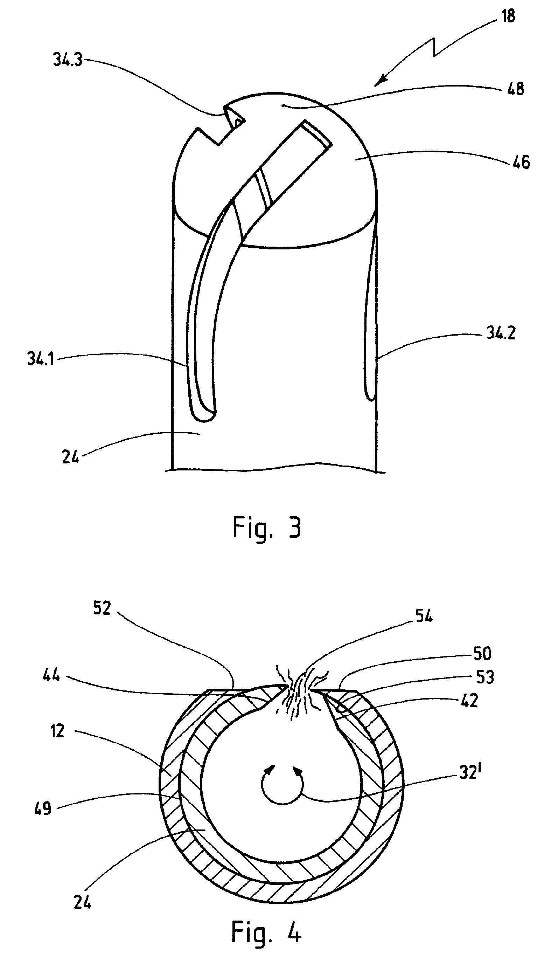

In Fig. 3 ist eine perspektivische Darstellung des distalen Endes 18 des Innenschafts 24 von Fig. 1 und Fig. 2 dargestellt.

Der Innenschaft 24 weist drei, umfänglich versetzt angeordnete, Innenschaftöffnungen 34.1, 34.2, 34.3 auf. Die Innenschaftöffnungen 34.1, 34.2, 34.3 erstrecken sich dabei bis zu einer distalen Endfläche 46, und zwar neben einem distalen Endpunkt 48, welcher den Scheitelpunkt des distalen Endes 18 bildet, so dass sie sich weder schneiden noch auf den distalen Endpunkt 48 zu laufen. Wie bereits vorangehend beschrieben, trägt diese Ausgestaltung zu einer Stabilitäts- und Steifigkeitserhöhung des Innenschafts 24 am distalen Ende 18 bei.

Es sei zudem angemerkt, dass der Außenschaft 12, welcher mit einem derartig ausgebildeten Innenschaft 24 zusammenwirkt, ebenfalls drei, umfänglich versetzt angeordnete, Außenschaftöffnungen 12.1, 12.2, 12.3 aufweisen kann, um die Schnittleistung weiter zu erhöhen.

Bei der in Fig. 4 dargestellten Schnittansicht entlang der Schnittlinie A-A von Fig. 2 weisen die erste und die zweite Innenschaftschneide 42, 44 der Innenschaftöffnung 34 jeweils einen keilförmige Querschnitt mit einer an einer Außenseite 49 des Innenschafts 24 angeordneten Keilspitze auf, um die Schnittaggressivität zu erhöhen. Zudem weist die Außenschaftöffnung 20 eine erste Außenschaftschneide 50 und eine zweite Außenschaftschneide 52 auf, welche ebenfalls keilförmig ausgebildet sind und jeweils eine auf Höhe der Innenfläche 53 des Außenschafts 12 angeordnete Keilspitze aufweisen. Dadurch kann die Schnittaggressivität weiter erhöht werden.

Ein Rotationspfeil 32' deutet dabei die Möglichkeit an, dass der Innenschaft 24 in beide Umdrehungsrichtungen bewegbar ist, so dass an beiden Außenschaftschneiden 50, 52 ein Schnittvorgang eines Gewebes 54 realisiert werden kann. Im dargestellten Ausführungsbeispiel rotiert der Innenschaft 24 im Uhrzeigersinn, so dass die zweite Innenschaftschneide 44 mit der ersten Außenschaftschneide 50 schneidend zusammenwirkt und das Gewebe 54 effizient abgetrennt werden kann.

Bei einer Rotation in die entgegengesetzte Richtung, z.B. während einer oszillierenden Bewegung, wirken analog die erste Innenschaftschneide 42 mit der zweiten Außenschaftschneide 52 schneidend zusammen und erzeugen den entsprechenden Schnittvorgang.

Der Einsatz des medizinischen Instruments 10 von Fig. 1 soll im Ablauf der Figuren 5A-C und 6A-C kurz erläutert werden.

Fig. 5 und Fig. 6 veranschaulichen den vorteilhaften Schnittverlauf und die verbesserte Schnittwirkung des erfindungsgemäßen Instruments aufgrund der gekrümmten, säbelförmigen Innenschaftschneide 44. Es sind jeweils drei Positionen A, B, C des Innenschafts 24 während des Durchlaufs der Innenschaftöffnung 34 durch die Außenschaftöffnung 20 gezeigt. Die Außenschaftöffnung 20 ist dabei ovalförmig (Fig. 5) bzw. länglich (Fig. 6) ausgebildet. Sobald die Innenschaftöffnung 34 die Außenschaftöffnung 20 passiert, bildet sich ein Saugbereich 56, welcher als schraffierte Fläche gekennzeichnet ist, und das Gewebe 54 wird in diesen Bereich durch den von der Saugquelle 40 erzeugten Saugstrom angesaugt. Durch die im Saugbereich 56 bestehende Saugwirkung wird das abzutrennende Gewebe 54 durch die Außenschaftöffnung 20 und die Innenschaftöffnung 34 eingesaugt. Durch das Vorbeilaufen der zweiten Innenschaftschneide 44 des Innenschafts 24 an der ersten Außenschaftschneide 50 des Außenschafts 12, wie es aus Fig. 5 und 6 ersichtlich ist, wird das eingesaugte abzutrennende Gewebe 54 abgetrennt. Das abgetrennte Gewebe 54 wird durch den Innenschaft 24 zum proximalen Ende des Instruments 10 hin abgesaugt.

Der während eines Schnittvorgangs zwischen der hinteren und somit schneidenden zweiten Innenschaftschneide 44 und der mit dieser zusammenwirkenden ersten Außenschaftschneide 50 entstehende Schnittwinkel 58 variiert während des gesamten Durchlaufs der Innenschaftöffnung 34 an der Außenschaftöffnung 12.

In Fig. 5A und Fig. 6A ist die "Saugphase" dargestellt, bei der lediglich Gewebe 54 angesaugt wird, da aufgrund des stumpfen Schnittwinkels 58 kaum eine Schnittwirkung erzielt wird. Wie aus Fig. 5B und Fig. 6B ersichtlich, beginnt die "Schnittphase" beim erfindungsgemäßen Instrument, im Vergleich zum einleitend beschriebenen Stand der Technik, bereits sehr früh und mit einem vorteilhaft spitzen Schnittwinkel 58. Die gekrümmte zweite Innenschaftschneide 44 wirkt dadurch sehr effektiv mit der ersten Außenschaftschneide 50 zusammen und durchtrennt dabei das Gewebe 54 säbelartig. Fig. 5C und Fig. 6C zeigen schließlich die "Endphase", bei der deutlich wird, dass der spitze Schnittwinkel während des Durchlaufs langsam und kontinuierlich geschrumpft ist, wodurch der Schnittverlauf einer sich langsam schließenden Schere, wie beispielsweise einer Amboss-Schere, imitiert wird und somit eine effizientere Schnittwirkung über eine längere Schnittphase gegenüber dem zu entfernenden Gewebe 54 erreicht wird.

## Patentansprüche

1. Medizinisches Instrument zum Schneiden von Gewebe (54), mit einem rohrförmigen Außenschaft (12), der im Bereich seines distalen Endes (18) zumindest eine Außenschaftöffnung (20) mit zumindest einer Außenschaftschneide (50, 52) aufweist, und mit einem rohrförmigen, um eine Rotationsachse (26) rotierbaren, in dem Außenschaft (12) aufgenommenen Innenschaft (24), der im Bereich seines distalen Endes (18) zumindest eine Innenschaftöffnung (34) mit zumindest einer Innenschaftschneide (42, 42) aufweist, die bei in Rotation versetztem Innenschaft (24) mit der zumindest einen Außenschaftschneide (50, 52) des Außenschafts (12) schneidend zusammenwirkt, wobei die zumindest eine Innenschaftöffnung (34) in einer parallel zur Rotationsachse (26) verlaufenden Projektionsebene (37) eine gekrümmte Längsachse (36) aufweist, **dadurch gekennzeichnet, dass** der Innenschaft (24) drei umfänglich versetzt angeordnete Innenschaftöffnungen (34, 34.1, 34.2, 34.3) aufweist, dass die Breite der Innenschaftöffnungen (34, 34.1, 34.2, 34.3), in einer Umfangsrichtung gesehen, geringer ist als der Abstand zwischen den Innenschaftöffnungen (34, 34.1, 34.2, 34.3), dass sich die drei Innenschaftöffnungen (34.1, 34.2, 34.3) bis zu einer distalen Endfläche (46) erstrecken und dass diese im Bereich der distalen Endfläche (46) neben einem distalen Endpunkt (48) verlaufen.

2. Medizinisches Instrument nach Anspruch 1, **dadurch gekennzeichnet, dass** die Längsachse (36) der Innenschaftöffnungen (34.1, 34.2, 34.3) einen kreisbogenförmigen Verlauf aufweist.

3. Medizinisches Instrument nach Anspruch 1 oder 2, **dadurch gekennzeichnet, dass** die zumindest eine Innenschaftschneide (42, 44) der drei Innenschaftöffnungen (34.1, 34.2, 34.3) einen keilförmigen Querschnitt mit einer Keilspitze aufweist, welche an einer Außenfläche (49) des Innenschafts (24) angeordnet ist.

4. Medizinisches Instrument nach Anspruch 3, **dadurch gekennzeichnet, dass** die zumindest eine Außenschaftschneide (50, 52) der zumindest einen Außenschaftöffnung (20) einen keilförmigen Querschnitt mit einer Keilspitze aufweist, welche an einer Innenfläche (53) des Außenschaftes (12) angeordnet ist.

5. Medizinisches Instrument nach Anspruch 1, **dadurch gekennzeichnet, dass** der Außenschaft (12) drei umfänglich versetzt angeordnete Außenschaftöffnungen (12.1, 12.2, 12.3) aufweist.

6. Medizinisches Instrument nach einem der Ansprüche 1 bis 5, **dadurch gekennzeichnet, dass** der Innenschaft (24) mittels eines Motors (30) in zwei Umlaufrichtungen bewegbar ist.

7. Medizinisches Instrument nach Anspruch 6, **dadurch gekennzeichnet, dass** der Innenschaft (24) über einen vordefinierten Winkelbereich oszillierend bewegbar ist.

8. Medizinisches Instrument nach Anspruch 7, **dadurch gekennzeichnet, dass** bei jeder Auslenkung des oszillierenden Innenschaftes (24) ein Schnittvorgang durchgeführt wird.

## Claims

1. Medical instrument for cutting tissue (54), with a tubular outer shaft (12) which, in the area of its distal end (18), has at least one outer shaft opening (20) with at least one outer shaft blade (50, 52), and with a tubular inner shaft (24) which is rotatable about a rotation axis (26), and which is received in the outer shaft (12) and, in the area of its distal end (18), has at least one inner shaft opening (34) with at least one inner shaft blade (42, 44) which, when the inner shaft (24) is moved in rotation, cooperates in a cutting action with the at least one outer shaft blade (50, 52) of the outer shaft (12), wherein the at least one inner shaft opening (34) has a curved longitudinal axis (36) in a plane of projection (37) running parallel to the rotation axis (26), **characterized in that** the inner shaft (24) has three circumferentially offset inner shaft openings (34, 34.1, 34.2, 34.3), further **in that** the width of the inner shaft openings (34, 34.1, 34.2, 34.3), seen in a circumferential direction, is smaller than the distance between the inner shaft openings (34, 34.1, 34.2, 34.3), further **in that** the three inner shaft openings (34.1, 34.2, 34.3) extend as far as a distal end face (46), and **in that**, in the area of the distal end face, these extend next to a distal endpoint (48).

2. Medical instrument of claim 1, **characterized in that** the longitudinal axis (36) of the three inner shaft openings (34.1, 34.2, 34.3) have a profile in the shape of an arc of a circle.

3. Medical instrument of claims 1 or 2, **characterized in that** the at least one inner shaft blade (42, 44) of the three inner shaft openings (34.1, 34.2, 34.3) has a wedge-shaped cross section with a wedge point that is arranged on an outer face (49) of the inner shaft (24).

4. Medical instrument of claim 3, **characterized in that** the at least outer shaft blade (50, 52) of the at least one outer shaft opening (20) has a wedge-shaped cross section with a wedge point that is arranged on an inner face (53) of the outer shaft (12).

5. Medical instrument of claim 1, **characterized in that** the outer shaft (12) has three circumferentially offset outer shaft openings (12.1, 12.2, 12.3).

6. Medical instrument of anyone of claims 1 through 5, **characterized in that** the inner shaft (24) can be moved in two directions of rotation by means of a motor (30).

7. Medical instrument of claim 6, **characterized in that** the inner shaft (24) can be moved in oscillation over a predefined angle range.

8. Medical instrument of claim 7, **characterized in that** a cutting procedure is carried out upon each excursion of the oscillating inner shaft (24).

## Revendications

1. Instrument médical destiné à couper des tissus (54), comportant une tige extérieure tubulaire (12) qui présente dans la zone de son extrémité distale (18) au moins une ouverture de tige externe (20) ayant au moins une lame de tige externe (50, 52), et comportant une tige interne (24) tubulaire pouvant pivoter autour d'un axe de rotation (26) et logée dans la tige externe (12), qui présente dans la zone de son extrémité distale (18) au moins une ouverture de tige interne (34) ayant au moins une lame de tige interne (42) qui, avec la tige interne (24) décalée en rotation, coopère par coupe avec la lame de tige externe (50, 52), au moins au nombre de une, de la tige externe (12), où l'ouverture de tige interne (34), au moins au nombre de une, présente, dans un plan de projection (37) s'étendant parallèlement à l'axe de rotation (26), un axe longitudinal incurvé (36), **caractérisé en ce que** la tige interne (24) présente trois ouvertures de tige interne (34, 34.1, 34.2, 34.3) disposées de façon décalée sur la périphérie, **en ce que** la largeur des ouvertures de tige interne (34, 34.1, 34.2, 34.3), vue dans une direction périphérique, est plus petite que l'écart entre les ouvertures de tige interne (34, 34.1, 34.2, 34.3), **en ce que** les trois ouvertures de tige interne (34.1, 34.2, 34.3) s'étendent jusqu'à une surface d'extrémité distale (46) et **en ce qu'**elles s'étendent dans la zone de la surface d'extrémité distale (46) à côté d'un point d'extrémité distal (48).

2. Instrument médical selon la revendication 1, **caractérisé en ce que** l'axe longitudinal (36) des ouvertures de tige interne (34.1, 34.2, 34.3) présente une allure en forme d'arc de cercle.

3. Instrument médical selon la revendication 1 ou 2, **caractérisé en ce que** la lame de tige interne (42, 44), au moins au nombre de une, des trois ouvertures de tige interne (34.1, 34.2, 34.3) présente une section transversale cunéiforme, avec une pointe cunéiforme disposée au niveau d'une surface externe (49) de la tige interne (24).

4. Instrument médical selon la revendication 3, **caractérisé en ce que** la lame de tige externe (50, 52), au moins au nombre de une, de l'ouverture de tige externe (20), au moins au nombre de une, présente une section transversale cunéiforme, avec une pointe cunéiforme disposée au niveau d'une surface interne (53) de la tige externe (12).

5. Instrument médical selon la revendication 1, **caractérisé en ce que** la tige externe (12) présente trois ouvertures de tige externe (12.1, 12.2, 12.3) disposées au niveau de la périphérie de façon décalée.

6. Instrument médical selon l'une des revendications 1 à 5, **caractérisé en ce que** la tige interne (24) peut être déplacée dans deux directions de circulation au moyen d'un moteur (30).

7. Instrument médical selon la revendication 6, **caractérisé en ce que** la tige interne (24) peut être déplacée de façon oscillante sur une plage angulaire prédéfinie.

8. Instrument médical selon la revendication 7, **caractérisé en ce qu'**un processus ou une opération de coupe est réalisé à chaque excursion de la tige interne oscillante (24).
